# EUROPÄISCHE PATENTSCHRIFT

(11) **EP 1 801 037 B1**
(45) Veröffentlichungstag und Bekanntmachung des Hinweises auf die Patenterteilung: **19.08.2009**
(21) Anmeldenummer: 06026679.8
(22) Anmeldetag: 22.12.2006
(51) Int. Cl.: B65D 88/34

(54) **Abdeckung für Güllebehälter, Biogasspeicher und dergleichen und Verfahren zum Abdecken eines Güllebehälters oder eines Biogasspeichers**
Cover for slurry tank, biogas storage and similar and method of covering a slurry tank or biogas storage
Recouvrement de réservoir pour purin, bio-gaz et similaire et méthode pour recouvrir un réservoir pour purin ou bio-gaz

(30) Priorität: 23.12.2005 DE 102005061841
(43) Veröffentlichungstag der Anmeldung: 27.06.2007
(73) Patentinhaber: agraferm technologies AG, 85276 Pfaffenhofen/Ilm (DE)
(72) Erfinder: Friedmann, Hans, Dr., 85296 Scheyern-Fernhag (DE)
(74) Vertreter: Ganahl, Bernhard

(56) Entgegenhaltungen:
- WO-A-03/018437
- CH-A5- 693 207
- DE-A1- 3 033 646
- US-A1- 3 120 902
- US-A1- 4 413 747
- US-A1- 4 437 987
- US-A1- 2002 070 152

## Beschreibung

Die Erfindung betrifft eine Abdeckung für Güllebehälter, Biogasspeicher und dergleichen.

Beim Umgang mit Gülle ist eine besondere Sorgfalt geboten. Dies gilt selbstverständlich auch für die Lagerung außerhalb von Ställen in sogenannten Güllebehältern. Diese Güllebehälter sind oft sogenannte Gülleteiche oder zylindrische nach oben offene Behälter. Eine permanente Beeinträchtigung der Umwelt besteht häufig in den luftgetragenen Emissionen von Güllebehältern. Dies ist einerseits der Geruch, wobei der typische Fäkalgeruch nicht unmittelbar gesundheitsschädlich ist, aber bei ungünstigen Standortbedingungen zu Belästigungen führen kann. Vor allem offene Güllelagerstätten in Ortslagen sind hier zu nennen. Emissionen aus der Gülle sind jedoch nicht nur unangenehm bezüglich ihres Geruchs, sie besitzen darüber hinaus weitreichende Auswirkungen auf das Ökosystem. Neben Methan (CH4) und Lachgas (N20) besitzt vor allem das ausgasende Ammoniak (NH3) ein erhöhtes Gefährdungspotential. Bei hohen Konzentrationen des Gases können Pflanzen direkt geschädigt wer den.

Gemäß einem Bericht der Landwirtschaftskammer Hannover mit dem Titel "Güllelagerung-Behälterabdeckungen" vom 04. Dezember 2001 sind in unmittelbarer Nähe von Stellen bis zu einer Entfernung von ca. 50 m Verfärbungen der Blätter und sogar das Absterben ganzer Pflanzen beobachtet worden. Wesentlich folgenschwerer sind die indirekten Auswirkungen der Ammoniakemissionen durch Säurebildung des Bodens (Umwandlung des NH3 in Salpetersäure), Verdrängung von für Pflanzen lebenswichtigen Ionen (Mg, Ca), Eutrophierung nährstoffarmer Gebiete und Förderung der Schwefeldioxidabscheidung. Obwohl die Bildung von Geruchs- und Ammoniakemissionen in keinem direkten Zusammenhang steht, ist eine wesentliche Reduzierung beider Emissionen mit der Abdeckung von Güllelagerstätten möglich. Alleine der behinderte Gasaustausch zwischen Gülleoberfläche und Atmosphäre bewirkt eine Verbesserung. Zum Schutz der Nachbarschaft vor Gerüchen und der Umwelt vor Schadgasen ist eine Abdeckung häufig erforderlich. Um einen solchen behinderten Gasaustausch zu gewährleisten sind verschiedene Formen von Abdeckungen bekannt.

Auf Rindergülle, die einen höheren Feststoffanteil besitzt, bildet sich in der Regel in kürzester Zeit eine dichte und feste natürliche Schwimmschicht, die über den Gesamtlagerungszeitraum eine Emissionsminderung von ca. 50 % bewirken kann. Eine solche Emissionsminderung ist jedoch häufig nicht ausreichend. Auf Schweine- und Geflügelflüssigmist tritt eine Schwimmdecke nur zeitweise oder gar nicht auf. Bei einer Mischgülle von Rindern und Schweinen ist eine Schwimmdecke nur zu erwarten, wenn die Rindergülle mindestens einen Anteil von 25 % ausmacht. Auch hierbei ist eine solche sich von selbst einstellende feste natürliche Schwimmschicht für die Emissionsminderung nicht ausreichend.

Darüber hinaus ist es bekannt, eine Strohhäckselschicht oder Schichten aus Blähtongranulat aufzubringen, wobei das Aufbringen dieser Schichten jedoch nicht einfach ist, und einen erhöhten Arbeitsaufwand bedeutet. Blähtongranulat ist zudem verhältnismäßig teuer.

Insbesondere wenn die entstehenden Gase weiter genutzt werden sollen, dies trifft insbesondere für Biogasbehälter zu, aber auch für Güllebehälter (wobei in vielen Fällen auch Biogasbehälter nicht unerhebliche Mengen von Gülle beinhalten) werden Abdeckungen insbesondere aus Folien eingesetzt. Im einfachsten Fall werden als Abdeckung von Behältern Schwimmfolien aus Kunststoffdichtungsbahnen eingesetzt, wobei bei ihnen insbesondere die Stabilität der Befestigungsösen zwischen Folienloch und Stahldrahtführung ständig zu prüfen ist.

Darüber hinaus gibt es die verschiedensten Varianten von Zeltdächern, wobei vorkonfektionierte Lösungen vorhanden sind, die freitragend oder auf systemabhängigen Unterkonstruktionen aufgebaut sind und ebenso wirksam sind wie konventionelle Dachkonstruktionen mit Holz und Dachpappe. Bei diesen Varianten sammelt sich zwischen Gülleoberfläche und Abdeckung ein Gasgemisch an. Um die Verpuffungs-und Explosionsgefahr zu reduzieren, gehören zu diesen Behältern immer Entlüftungsöffnungen.

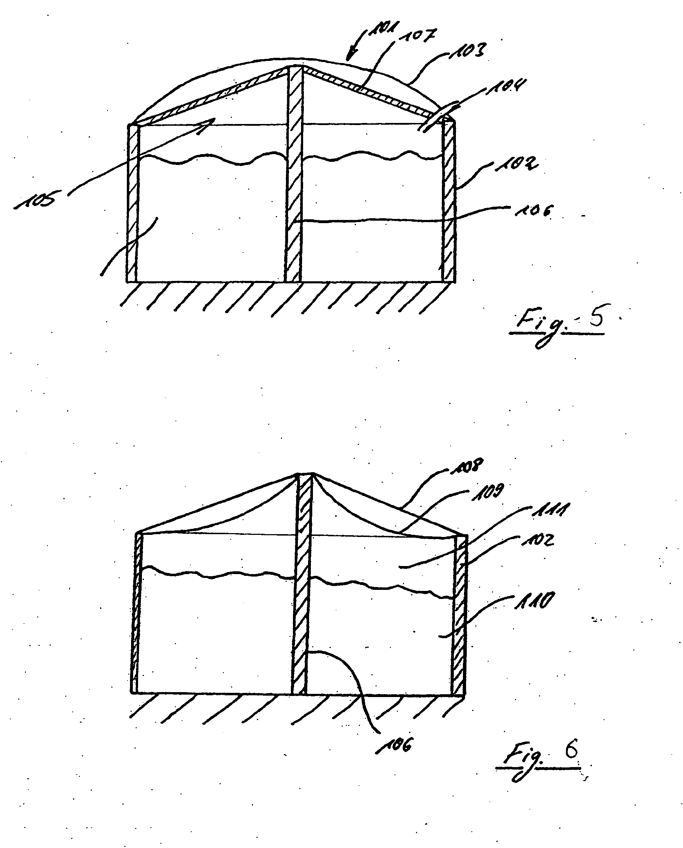

Beispielsweise ist es bekannt (Fig. 5), ein Dach 101 über einen Güllebehälter oder Gasspeicher 102 zu spannen, bei dem eine äußere elastische Folie 103 vorhanden ist und zudem eine Gasableitung 104 vorhanden ist, wobei die äußere Folie 103 auf einem Unterbau 105 ruht, der aus einer in der Gülle ruhenden Stütze 106 und von der Stütze zu den Außenwandungen radial verlaufenden Seilen oder Sparren 107 ausgebildet ist. Hierbei ist von Nachteil, dass die Streben bzw. Stützen häufig aus Holz sind und relativ schnell verrotten. Darüber hinaus machen derartige Konstruktionen ein Investitionsvolumen von etwa 25.000 € aus, was relativ teuer ist.

Bei einer weiteren Ausführungsform im Stand der Technik (Fig. 6) besitzt ein beispielsweise zylindrischer Güllebehälter 102 eine zentrale Stütze 106, wobei von der Stütze radial nach außen eine erste Wetterschutzmembran 108, beispielsweise aus einem entsprechenden Kunststoff nach außen zu den Wänden festgespannt ist, so dass keine weitere Seil- oder Sparrenkonstruktion vorhanden ist. Um einen Volumenausgleich zu gewährleisten ist im Behälter unterhalb der Wetterschutzmembran eine Innenmembran 109 vorhanden, welche gasdicht ist und zwischen der Flüssigkeit 110 und der gasdichten Innenmembran einen Gasraum 111 begrenzt. Der Druck im Gasraum 111 beträgt beispielsweise 2 bis 3 mbar. Hierbei ist von Nachteil, dass zwei Membranen benötigt werden, und zudem die Membranen außen an den Behälterwandungen gasdicht befestigt werden müssen, was einen gewissen Aufwand erfordert, da hier auch eine Druckdichtigkeit gegeben sein muss. Zudem werden hier Investitionsvolumina von 45.000 € notwendig, so dass auch eine solche Vorrichtung sehr teuer ist.

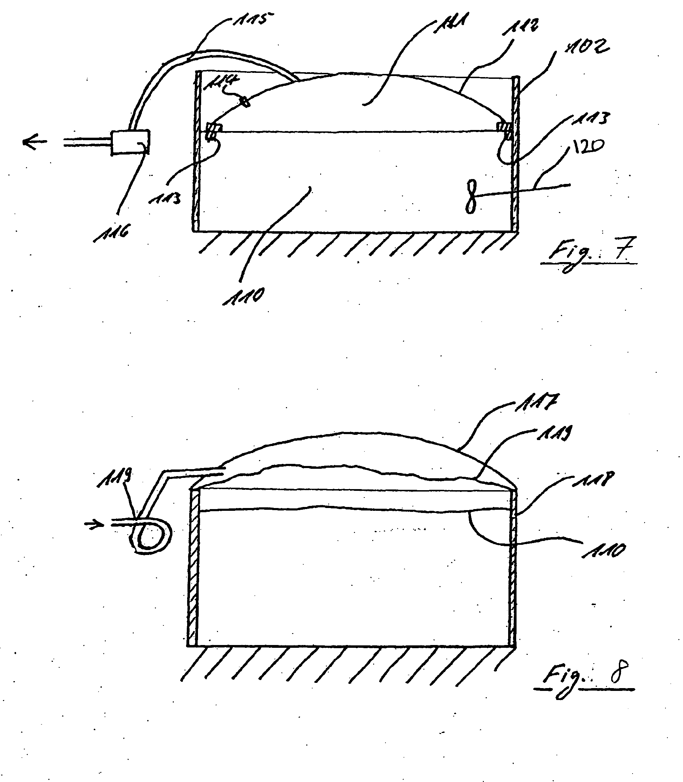

Darüber hinaus ist es bekannt (Fig. 7) - insbesondere bei Gasspeichem für Biogasanlagen - eine Membran 112 auf die Flüssigkeit 110 aufzulegen, wobei außenseitig die Membran 112 - die vorzugsweise die gesamte Flüssigkeit überspannt - mit einem Holzring 113 auszubilden, der auf der Flüssigkeit 110 schwimmt. Durch den entstehenden Gasdruck bläht sich die Membran bis zu einem gewissen Grade auf, wobei ein Drucksensor 114 vorhanden ist und eine Gasableitung 115, die zu einem Verdichter 116 führt, der das entstehende Gas verdichtet und für die Weiterverarbeitung komprimiert. Bei dieser Vorrichtung ist von Nachteil, dass bei einem Überdruck, der über dem Nenndruck von 1 mbar liegt, Gas unter dem Rand des schwimmenden Ringes austreten kann. Ein Hauptnachteil ist jedoch, dass zum Auflegen der Membran auf einen Gülle- bzw. Biogasbehälter bei den üblichen Durchmessern von 20 m ein 250-t-Mobilkran benötigt wird, so dass die Montagekosten jeweils deutlich über 5.000 € liegen. Die Vorrichtung alleine verursacht Kosten von 30.000 €, so dass auch hier ein sehr hohes Investitionsvolumen vorhanden ist. Ein weiterer Nachteil ist, dass bei einem besonders niedrigen Güllestand bzw. Flüssigkeitsstand der Holzring mit dem obligatorischen Rührer 120 kollidieren kann. Ferner ist von Nachteil, dass eine solche Vorrichtung außerordentlich windanfällig ist, dies betrifft sowohl die Montage als auch die im Betrieb befindliche Abdeckung.

Bei einer weiteren aus dem Stand der Technik bekannten Abdeckung (Fig. 8) ist eine Außenmembran 117 fest mit den Wandungen 118 des Behälters verbunden und mit einem Gebläse 119 wird zwischen der Flüssigkeit 110 und der Außenmembran 117 ein Druck von beispielsweise 10 mbar ausgebildet, so dass sich die Außenmembran 117 nach außen aufbläht. Dies funktioniert analog zu beispielsweise freitragenden Tennishallen, die auf einem ähnlichen Prinzip beruhen. Für einen Volumenausgleich sorgt auch hier eine Innenmembran 119, wobei auch hier erhebliche Investitionen notwendig sind, da ein erhebliches Dichtigkeitsproblem auf Grund der hohen Zugspannung im Bereich der Anbindung der Außenmembran an die Behälterwandung besteht. Ferner wird permanent Strom für das Gebläse verbraucht.

Aus der WO 83/03884 A1 ist eine Abdeckung für nach oben offene Behälter bekannt, die sich flächig über den Behälterinhalt erstreckt und fest mit der Behälterwand verbunden ist. Die Abdeckung weist einen Aussteifungsring auf der der gasdichten Folienkonstruktion ihre Form verleiht und durch das erzeugte Gas angehoben wird. Die Erzeugung des Gasdrucks unterhalb der kugelkalottenartig ausgebildeten Folienkonstruktion kann entweder durch den Aussteifungsring selbst oder durch an ihm befestigte Gewichte erzeugt werden.

Aus der JP 08-099096 A geht ein Methanisierungsbehälter hervor, auf dem, ein auf Schienen gelagerter Methanspeicher sitzt. Der Methanspeicher ist gegenüber dem Methanisierungsbehälter abgedichtet. Der Methanspeicher befindet sich in einem abgedichteten Wasserbehälter, um Erdbebensicherheit zu gewährleisteten.

Aus der WO 02/48311 A2 ist ein System bekannt, um aus Behältern für organische Abfälle anaerobe Faulbehälter zu machen. Das System beinhaltet eine aufblasbare Dachstruktur, die auf vielen Behältern angebracht werden kann, um diese gegen die Umwelt abzudichten. Die Dachstruktur besteht aus einer inneren gasundurchlässigen Membran, die mit dem organischen Abfall steigt und sinkt. Zudem gibt es eine Gasentnahmeeinheit, um das Gas unter der undurchlässigen Membran zu entnehmen. Das Gas wird dann zwischen der inneren und der äußeren Dachstruktur gespeichert.

Aus der FR 2 469 455 A1 geht eine Abdeckung hervor, die aus einem aufblasbaren Körper besteht und durch das Aufblasen eine bestimmte Form verliehen bekommt. Diese Abdeckung ist fest mit einer Bodenplatte verbunden, auf der ein festes Haufwerk aufliegt. Über ein Ventil auf der Seite kann die Abdeckung aufgepumpt werden und über eine Leitung mit Rückschlagventil kann das Gas entnommen werden.

Aus der CH 693 207 A5 geht eine Einrichtung zur Eindämmung der Geruchsbildung einer in einem über einer lagernden Flüssigkeit offenen Behälter hervor. Die Einrichtung ist eine Abdeckvorrichtung, die aus einer starren oder flexiblen Membran schwimmfähig ausgebildet ist. Die Abdeckvorrichtung weist mit der Membran verbundene, zumindest teilweise tragende, Schwimmelemente auf. Die Schwimmelemente können aus Kunststoff ausgebildet sein. Die Abdeckvorrichtung kann aus einer doppelten Folie ausgebildet sein, bzw. kann die Membran zusätzliche Kammern aufweisen, die Luft enthalten, um die Abdeckvorrichtung schwimmfähig auszugestalten. Die Membran der Abdeckvorrichtung ist im Behälter gegen Verdrehen oder Absinken arretiert. Die Arretierung erfolgt über eine Führungsanordnung die den äußeren Randbereich der Membran mit einer Behälterwand verbindet.

In der US 3 120 902 A1 ist eine schwimmende Abdeckstruktur beschrieben, (die auf einem Behälter vollflächig aufliegt). Die Abdeckstruktur ist kreisförmig ausgebildet und weist mehrere aufblasbare Kreisegmente auf. Im Zentrum der Kreissegmente ist ein Mittelteil mit einem Gasspeicher zur Aufnahme von Gasen aus dem Behälter ausgebildet. Über einen am Mittelteil angeordneten Flansch werden die Innenbereiche der Kreissegmente aufgenommen. Am äußeren Umfang der Kreissegmente ist ein Aussteifungsring angeordnet. Der Aussteifungsring ist aus Metall ausgebildet und weist einen Flansch auf, um die äußeren Enden der Kreissegmente aufzunehmen. Entlang des äußeren Umfangs des Aussteifungsring der Abdeckstruktur sind Rollen angeordnet, die die Abdeckstruktur mit der Behälterwand verbinden und um die Reibung zwischen der Behälterwandung und der Abdeckstruktur zu reduzieren. Es ist vorgesehen den Aussteifungsring innerhalb des entleerten Behälters aufzubauen und den Rest der Abdeckstruktur in unaufgeblasenem Zustand in einen Behälter einzubringen, um ihn dort mit dem Aussteifungsring zu verbinden und aufzublasen. Durch das Aufblasen soll die Abdeckstruktur ihre vorbestimmte Form einnehmen.

Aus der DE 30 33 646 A1 geht eine Abdeckung für ein Abwasser beinhaltendes Becken hervor. Die Abdeckung ist als Schwimmkörper mit einem die Oberfläche des Abwassers überspannenden Abdeckteil ausgebildet. Beim Überdecken des Beckens mit der Abdeckung und beim Entfernen der Abdeckung soll eine leichte Handhabung gegeben sein, so dass die Arbeiten mit geringer Kraft durchgeführt werden können. Der Schwimmkörper der Abdeckung kann aufblasbar in Form eines geschlossenen Rings mit einer Außenhülle aus einer flexiblen Kunststofffolie ausgebildet sein. Die Abdeckung soll selbst tragend und eigenstabil ausgebildet sein, wobei eine stabile und feste Schwimmlagerung der Abdeckung im Becken ohne zusätzliche Befestigungsmittel möglich ist.

Aufgabe der Erfindung ist es, eine Abdeckung für Güllebehälter und Gasspeicher von Biogasanlagen zu schaffen, die leicht zu montieren ist, eine hohe Stabilität aufweist und kostengünstig ist.

Die Aufgabe wird mit einer Abdeckung mit den Merkmalen des Anspruchs 1 gelöst.

Vorteilhafte Weiterbildungen sind in Unteransprüchen angegeben.

Eine erfindungsgemäße Abdeckung für Güllebehälter und Gasspeicher von Biogasanlagen besitzt einen falt-und aufblasbaren Schwimmkörper, der sich flächig über die flüssige Gülle oder das flüssige gaserzeugende Medium eines Gasspeichers erstreckt und auf diesem frei schwimmt. Die Abdeckung ist ein aufblasbarer und luftmatratzenartig ausgebildeter Schwimmkörper, der sich über die gesamte Fläche der Abdeckung erstreckt und der durch das Aufblasen selbstständig entfaltbar ist und eine vorbestimmte Form und Eigensteifigkeit einnimmt, und der im nicht aufgeblasenen Zustand zusammmenfaltbar ist.

Hierbei ist von Vorteil, wenn sich der Schwimmkörper nach dem Auflegen der Abdeckung auf ein Güllebad aufblasen lässt und entsprechend aufschwimmt.

Die Abdeckung ist dabei so ausgebildet, dass sie der Innenkontur eines entsprechenden Behälters oder eines entsprechenden Gasspeichers derart entspricht, dass sie die im Behälter enthaltene Flüssigkeit von oben her fast vollständig abdeckt, und mit geringem Spiel im Behälter frei schwimmt.

Die Äbdeckung ist ein luftmatratzenartige ausgebildeter Körper, der je nach Behälter rund oder eckig ist.

Erfindungsgemäß besitzt die Abdeckung im Randbereich unterhalb des Schwimmkörpers Gewichte, die die Abdeckung auf der Flüssigkeit halten.

Bei einer Ausführungsform sind somit im Randbereich des luftmatratzenartigen Körpers umlaufend beispielsweise durchgehend umlaufend oder beabstandet Schnüre oder Folienbahnen mit an deren Ende befestigten Gewichten vorhanden. Anstelle von einzelnen Gewichten kann auch eine durchgehende oder unterbrochene Folienbahn im Randbereich verlaufen, wobei die Folie eine Schwerschichtfolie ist, die neben einem Kunststoff schwere Zuschlagstoffe, wie Schwermineralien enthält. Bei einer Verwendung als Abdeckung für einen Güllebehälter kann es ausreichend sein, die Abdeckung flächig auszubilden, wobei die Abdeckung nahezu vollflächig auf der Gülle liegt.

Der Schwimmkörper ist ein aufblasbarer Hohlkörper, der Abdeckung die Eigensteifigkeit in aufgeblasenem Zustand verleiht.

Der Schwimmkörper kann aus mehreren einzelnen Schwimmkörpern ausgebildet sein. Die einzelnen aufblasbaren Schwimmkörper können als separate Schwimmkörper und separat aufblasbare Schwimmkörper ausgebildet sein. Es ist jedoch auch möglich, alle Schwimmkörper derart kommunizierend auszubilden, dass sie gemeinsam aufblasbar sind. Dies ist insbesondere dann zweckmäßig, wenn eine permanent angeschlossene Pumpe verwendet wird, die den Druck in den Schwimmkörpern konstant hält.

Die Abdeckung kann mit einer Beschwerungseinrichtung versehen sein, die außenseitig bzw. randseitig an der Abdeckung angeordnet ist. Diese Beschwerungseinrichtung ist insbesondere aus Folienbahnen ausgebildet, die wie die Flüssigkeit seitlich am Rand der Abdeckung angeordnet sind und sich vom Rand der Abdeckung nach unten erstrecken. An der bzw. den Folienbahnen kann ein zusätzliches Gewichtselement vorgesehen sein.

Die Abdeckung ist vorzugsweise am Umfang mit einem durchgehenden Folienstreifen versehen, der sich nach unten erstreckt. Dieser Folienstreifen kann Bestandteil der Beschwerungseinrichtung sein.

Die Folie, aus der bzw. die Schwimmkörper ausgebildet sind, weist vorzugsweise eine Dicke von 1 bis 3 mm bei einem Flächengewicht von 2 bis 5 kg/m² auf. Eine solche Folie kann auch als außenseitige Abdeckfolie verwendet werden.

Die Erfindung wird anhand einer Zeichnung beispielhaft erläutert. Es zeigen dabei:
- Figur 1:: eine Ausführungsform der erfindungsgemäßen Abdeckung in einer schematischen geschnittenen Ansicht;
- Figur 2:: eine Detailansicht der Abdeckung nach Fig. 1 an einem Randbereich;
- Figur 3:: den Randbereich einer Abdeckung nach Fig. 1 in einer seitlichen Ansicht einer ersten Ausführungsform;
- Figur 4:: den Randbereich einer erfindungsgemäßen Abdeckung in einer zweiten Ausführungsform;
- Figur 5:: eine Abdeckung nach dem Stand der Technik;
- Figur 6:: eine weitere Abdeckung nach dem Stand der Technik;
- Figur 7:: wiederum eine weitere Abdeckung nach dem Stand der Technik;
- Figur 8:: eine weitere Abdeckung nach dem Stand der Technik.

Eine erfindungsgemäße Abdeckung 1 dient der Abdeckung einer in einem Behälter 2 enthaltenen Flüssigkeit 3.

Der Behälter 2 kann ein in die Erde eingelassener Behälter 2 sein, oder ein Behälter 2, der mit Wänden 4 errichtet wurde, die einen Raum umschließen.

Die Abdeckung 1 besitzt eine äußere Kontur, die der Innenkontur des Behälters 2 im Wesentlichen entspricht und vorzugsweise einen Durchmesser bzw. Abmessungen besitzt, die im Wesentlichen dem Innendurchmesser bzw. den inneren Abmessungen des Behälters 2 entsprechen. Die Abdeckung 1 ist somit bei einem runden Behälter 2 grob scheibenförmig bzw. bei einem rechteckigen oder quadratischen Behälter 2 quadratisch oder rechteckig flächig ausgebildet und so ausgebildet, dass ein innerhalb der Wandungen 4 befindliches Volumen möglichst vollständig mit geringem Abstand zu den Wandungen 4 abgedeckt wird.

Die Abdeckung 1 besitzt zumindest einen Hohlkörper 5, der auf einer Flüssigkeit 3 schwimmen kann. Vorzugsweise ist der Hohlkörper bzw. Schwimmkörper 5 so ausgebildet, dass er mit einem Gas befüllbar ist und deshalb aufblasbar und ablassbar ausgebildet ist.

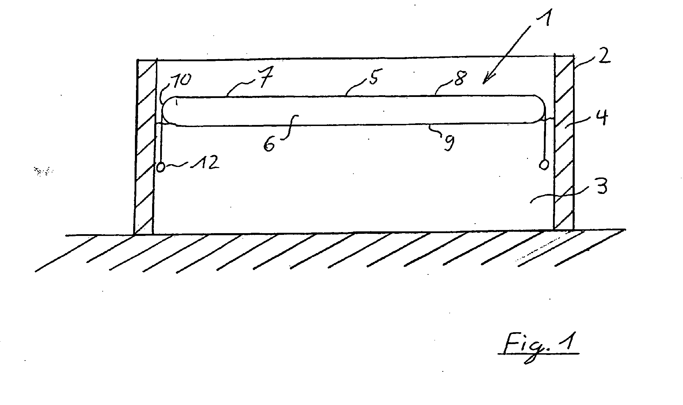

Im einfachsten Fall ist die Abdeckung 1 mit einem Hohlkörper 5 ausgebildet, der nach Art einer Luftmatratze flächig ausgebildet ist und einen äußeren Umfang oder einen Durchmesser besitzt, der im Wesentlichen dem Innenumfang bzw. Innendurchmesser des Behälters entspricht (Fig. 1). Hierbei liegt der Hohlkörper 5 flächig auf der Flüssigkeit 3 auf. Der Hohlkörper 5 weist eine einen Gasraum 6 umschließende Hülle 7 auf. Die Hülle 7 ist aus einer Oberwandung 8 und einer Unterwandung 9 und aus einer umlaufenden Seitenwandung 10 ausgebildet, die eine Verbindung zwischen der Oberwandung 8 und der Unterwandung 9 bildet. Der Hohlkörper weist mehrere separate Luftkammern auf, die durch Zwischenwandungen (nicht gezeigt) voneinander getrennt sind. Der Hohlkörper 5 liegt mit seinem Eigengewicht auf der Flüssigkeit 3 auf, so dass der Bereich, über den sich die Abdeckung 1 erstreckt, nach oben dicht, insbesondere gas- und flüssigkeitsdicht abgeschlossen ist.

Die Abdeckung bedeckt die Flüssigkeit, bis auf einen kleinen Randbereich, nahezu vollflächig. Auf diese Weise wird die Geruchsbildung auf ein Minimum reduziert. Durch Befüllen des Gasraums 6 mit Luft oder einem anderen Gas nimmt die Abdeckung 1 eine vorgegebene steife Form ein und behält diese.

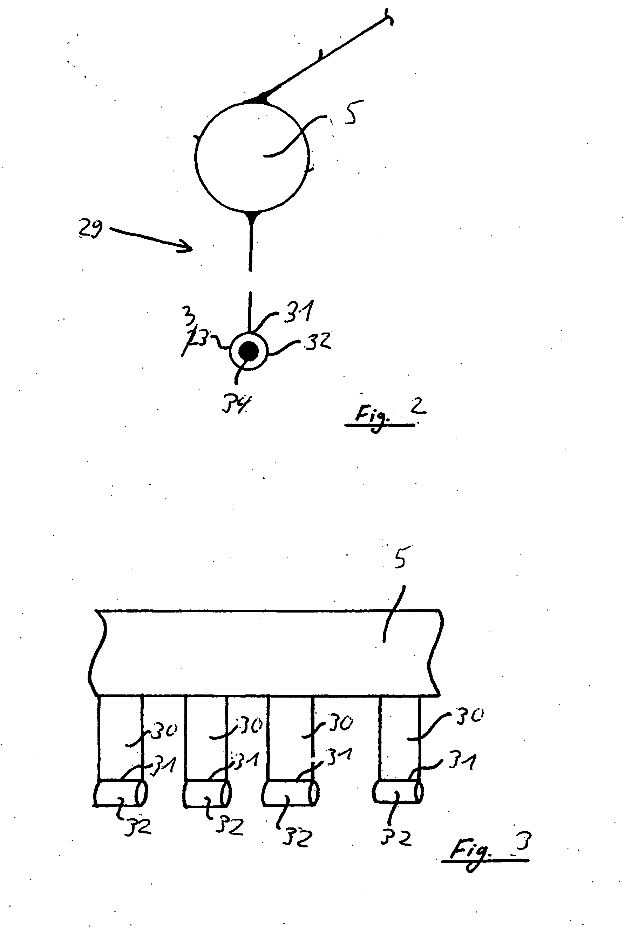

Die Abdeckung 1 gemäß Fig. 1 kann durch ein zusätzliches Gewicht 12 auf der Flüssigkeit gehalten werden. Geeignete Ausführungsformen des Gewichts werden in Form einer Beschwerungseinrichtung 29 (Fig. 2, Fig. 3, Fig. 4) unten noch ausführlich beschrieben.

Die Eigensteifigkeit der Abdeckung 1 kann durch Versteifungselemente (nicht gezeigt) noch verbessert werden. Die Versteifungselemente können als steife Schwimmkörperteile oder zusätzliche Versteifungselemente insbesondere radial verlaufende ausgebildet sein. Derartige Versteifungselemente sind beispielsweise umlaufende Schwimmkörperteile, die sich über einen bestimmten Abschnitt z.B. 1 bis 2 m erstrecken. Die Gesamtheit der Schwimmkörperteile bildet einen Schwimmkörper.
Die Länge der Schwimmkörperteile ist begrenzt, damit der Schwimmkörper und damit auch die Abdeckung 1 faltbar und zusammenlegbar ist.

Da die gesamte Abdeckung 1 im nicht aufgeblasenen Zustand zusammengefaltet werden kann und dadurch nur eine geringe Windangriffsfläche bietet, kann sie von einem kleinen Bagger angehoben und auf dem Flüssigkeitsspiegel abgelegt werden.
Es ist nicht notwendig, hierzu einen 250-Tonnen-Kran zu verwenden. Zum Anheben ist vorzugsweise eine nach außen weisende Öse im Radiusmittelpunkt bzw. geometrischen Mittelpunkt vorhanden.

Bei einer bevorzugten Ausführungsform ist an dem Hohl- bzw. Schwimmkörper 5 eine Pumpe angeschlossen, welche die Gesamtkonstruktion 1 erstmalig mit Luft bzw. Gas befüllt und zudem den für die Aussteifung der Konstruktion 1 erforderlichen Innendruck hält. Hierzu ist gegebenenfalls ein Drucksensor vorhanden, welcher die Pumpe steuert. Vorzugsweise ist die Pumpe eine vom Stromnetz unabhängige Pumpe, die insbesondere über ein Solarmodul mit elektrischer Energie versorgt wird, so dass es ein autarkes System darstellt.

Außenseitig bzw. randseitig besitzt die Abdeckung 1 im Bereich des Hohl- bzw. Schwimmkörpers 5 eine Beschwerungseinrichtung 29, welche die Abdeckung 1 auf dem Flüssigkeitsspiegel hält und gegen von außen angreifende Windlasten sichert.

Die Einrichtung 29 (Fig. 2) besteht beispielsweise aus Folienbahnen 30 (Fig. 3), die unterseitig, d. h. flüssigkeitsseitig, an dem Schwimm- bzw. Hohlkörper 5 angeordnet sind und sich der Schwerkraft entsprechend in die Flüssigkeit 3 hinein erstrecken. An einem freien Ende 31 der Folienbahnen 30 ist ein Gewicht 32 angeordnet, wobei das Gewicht 32 beispielsweise aus einem Kunststoffhohlkörper 33 besteht, in dessen Mitte ein Metallgewicht 34 angeordnet ist. Die Folienbahnen 30 sind bezüglich des axialen Verlaufs des Hohl- oder Schwimmkörpers 5 voneinander beabstandet und besitzen eine festgelegte Breite, wobei das Gewicht 34 beispielsweise zylinderartig ausgebildet ist (Fig. 3). Anstelle von Folienbahnen sind auch Schnüre mit Gewichten geeignet (nicht gezeigt).

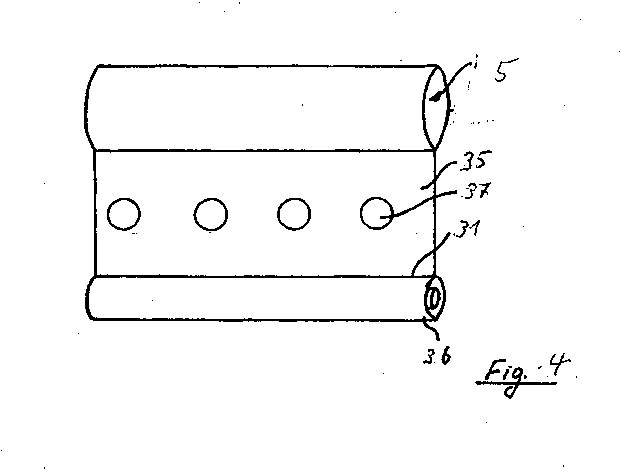

Bei einer weiteren vorteilhaften Ausführungsform (Fig. 4) ist anstelle mehrerer Folienstreifen 30 ein durchgehender Folienstreifen 35 vorhanden, der sich umlaufend vom Hohl- bzw. Schwimmkörper 5 nach unten erstreckt. An seinem unteren freien Ende bzw. seiner unteren umlaufenden freien Längskante 31 besitzt der Folienstreifen 35 einen umlaufenden Gewichtsring 36. Anstelle eines umlaufenden Gewichtsringes 36 oder einzelner Gewichte 32 ist es auch möglich, die Folienstreifen 30 bzw. die umlaufende Folie 35 als dicke Schwerfolie auszubilden, wobei diese Folie neben dem die Matrix bzw. tragende Struktur ausbildenden Kunststoff- bzw. Polymermaterial sogenannte Schwerzuschläge, insbesondere metallische Schwerzuschläge und/oder mineralische Schwerzuschläge, wie Eisenoxide und/oder Bariumsulfat oder ähnliche Schwerzuschläge enthält.

Sollte der Gasdruck durch aufsteigende Gase unterhalb der Abdeckung zu hoch werden und entsprechend Gas versuchen unterhalb des Schwimmkörpers 5 zu entweichen, kann dies entweder durch die Zwischenräume zwischen den Folienstreifen 30 oder Entgasungsöffnungen 37, beispielsweise Entgasungslöcher 37 in der Folienbahn 35 geschehen. Durch die Zwischenräume oder die Entgasungsöffnungen 37 austretendes Gas kann zwischen dem Schwimm- oder Hohlkörper 5 und einer Behälterwandung 4 vorhandenen Spalten austreten.

Vorzugsweise ist die gesamte Abdeckung 1 einstückig aus einem entsprechenden geeigneten Kunststoff hergestellt. Insbesondere finden die üblichen Kunststoffe Anwendung, die auch bei der Herstellung von Schlauchbooten und insbesondere hochwertigen Schlauchbooten angewendet werden. Dies schließt faserverstärkte oder durch textile Einlagen verstärkte Kunststoffe mit ein, die geeignet sind, entsprechende Innendrücke im Schwimmkörper 5 dauerhaft zu halten und gegen Belichtung und Bewitterung stabil sind. Die Folie hat beispielsweise eine Dicke von 1 bis 3 mm bei Flächengewichten bis zu etwa 3 kg/m².

Die Abdeckung 1 wird beispielsweise in nicht aufgeblasenem Zustand angeliefert und kann in einfachster Weise, beispielsweise mit einem Radlader in den Behälter 2 und auf die Flüssigkeit 3 aufgelegt werden. Anschließend wird die Konstruktion 1 mit der vorhandenen Pumpe aufgeblasen, wodurch sich die Konstruktion 1 in einfacher Weise von selbst ausrichtet und sich im Behälter 2 zentriert und sich entsprechend einschwimmt. Hierdurch ist die Montage besonders einfach und kann auch ohne Fachpersonal und ohne besondere Kranvorrichtungen durchgeführt werden.

Um eine Zentrierung zu erreichen und die Abdeckung trotzdem noch relativ gut handhaben zu können, kann die Abdeckung 1 über unterschiedliche Luftkammern verfügen. Hierzu ist es dann jedoch notwendig, dass jede Luftkammer über einen separaten Befüllungsschlauch verfügt, der an die Pumpe angeschlossen ist. Dies bedeutet einen Mehraufwand, wobei jedoch von Vorteil ist, dass beim Auftreten eines Lecks in einer der Luftkammern nicht die gesamte Konstruktion unbrauchbar wird. So ist es beispielsweise möglich, dass bei einem Erschlaffen einer Luftkammer die anderen Luftkammern die Vorrichtung 1 schwimmend halten und umgekehrt.

Vorzugsweise wird die Pumpe elektrisch betrieben, wobei neben der Pumpe ein Speichermedium für elektrischen Strom (Akkumulator, nicht gezeigt), sowie ein Elektrosolarmodul vorhanden sind, so dass die Pumpe Tag und Nacht autark betrieben werden kann. Zudem ist ein Drucksensor vorhanden, der die Pumpe derart steuert, dass sie nur im Falle eines kritischen Drucks derart lange betätigt wird, bis ein Differenzdruck erreicht wird, der für die Betriebsfähigkeit der Abdeckung 1 ausreichend ist. Vorteilhafterweise besitzen die Pumpe und/oder die Steuerung der Pumpe (nicht gezeigt) eine Warneinrichtung derart, dass, wenn die Pumpe nicht mehr in der Lage ist, den Druck aufrecht zu erhalten, der für den Betrieb der Abdeckung erforderlich ist (beispielsweise bei Auftreten eines Lecks oder einem Schaden an der Pumpe), der Betreiber eine Wammeldung gleich welcher Art erhält, um Gegenmaßnahmen zu ergreifen.

Um einen Spalt zwischen dem Schwimmkörper 5 bzw. der Einrichtung 29 und der Behälterwandung 4 sicherzustellen sowie die Reibung zu vermindern, können radial umfänglich an der Einrichtung 29 und/oder dem Schwimm- bzw. Hohlkörper 5 Gleitschienen vorhanden sein, die nach außen weisen. Derartige Gleitschienen können aus dem gleichen Werkstoff hergestellt sein, wie die Folien, z. B. aus Polyolefinen oder aus Teflon oder aus Holz.

Bei dieser Ausführungsform ist die Beschwerungseinrichtung 29 als ein durchgehender Folienstreifen 35 ausgebildet, der im unteren Bereich, der benachbart zum Gewichtsring 36 angeordnet ist, mit Entgasungslöchern 37 versehen ist. Das Biogas sammelt sich unter der Abdeckung 1, wobei sich der Schwimmkörper 5 vom Flüssigkeitsspiegel der Flüssigkeit 3 abheben kann bis die Entgasungslöcher 37 auf die Höhe des Flüssigkeitsspiegels gelangen, so dass weiteres Biogas durch die Entgasungslöcher 37 entweicht. Durch das Vorsehen der Gleitschienen wird ein Verkannten der Abdeckung 1 innerhalb des Behälters 2 verhindert und das Anheben bzw. Absenken der Abdeckung 1 erleichtert.

Die erfindungsgemäße Abdeckung .1 kann auch mit nicht aufblasbaren Schwimmkörpem, die z.B. aus Polystyrol oder anderen geschäumten Kunststoffen oder Holz ausgebildet sind, versehen sein. Derartige Schwimmkörper stellen sicher, dass die Abdeckung 1 beim Auflegen auf die Flüssigkeit 3 nicht in dieselbe eintaucht. Für die Erfindung ist es jedoch von Bedeutung, dass diese nicht aufblasbaren Schwimmkörper keinen steifen umlaufenden Ring ausbilden, sondern im Vergleich zu den Gesamtabmessungen der Abdeckung 1 relativ kleine Abschnitte bilden, so dass die Abdeckung 1 im nicht-aufgeblasenen Zustand relativ klein zusammenlegbar ist. Durch das Aufblasen der Abdeckung nimmt diese dann, wie es oben erläutert ist, die vorbestimmte Form an, womit eine zuverlässige Abdeckung der Flüssigkeit 3 gewährleistet wird.

Die Abdeckung 1 ist in nicht aufgeblasenem Zustand relativ klein verpackbar und mit einem herkömmlichen kleineren Bagger oder einem Frontlader ohne weiteres auf einen Güllesee, einen Güllebehälter oder einen Gasspeicher auflegbar. Durch das Aufblasen des zumindest einen Hohl- bzw. Schwimmkörpers 5 entfaltet sich die Abdeckung 1, nimmt ihre vorbestimmte Form ein und breitet sich in den unterschiedlichen beschriebenen Weisen über einer Flüssigkeit 3 aus, die es zuverlässig nach oben abdeckt. Die Abdeckung besitzt dann eine ausreichende Eigensteifigkeit, so dass sie nicht selbstständig durch Umwelteinflüsse zusammengefaltete werden kann, sondern auf Dauer sicher die Flüssigkeit abdeckt.

Die Folienstärke für die Folien beträgt beispielsweise 2 bis 3 mm bei einem Flächengewicht von 3 kg pro m². Der Durchmesser einer derartigen Abdeckung kann beispielsweise 18 m betragen, wobei dann ein Gewicht von 1,5 t besteht.

Bei der Erfindung ist von Vorteil, dass eine derartige Abdeckung nach Art einer Luftmatratze relativ einfach und kostengünstig herstellbar ist. Darüber hinaus ist von Vorteil, dass eine solche Abdeckung in den meisten Fällen ohne großen apparativen Aufwand selbst montiert werden kann und sich durch das Aufblasen selbst zentriert, einschwimmt und auf der Flüssigkeit 3 anordnet.

Darüber hinaus ist von Vorteil, dass keine Holzkonstruktion enthalten ist, die gegebenenfalls verrotten kann. Ferner ist von Vorteil, dass die Unterhaltskosten gering sind, da nach dem einmaligen Aufblasen mit Luft eine relativ kleine Pumpe, beispielsweise die solarbetrieben ist, ausreichen kann, um den Luftdruck zu halten. Selbst diese Pumpe kann überflüssig sein, wenn der Luftdruck regelmäßig kontrolliert wird und mit handelsüblichen Pumpen oder Gasflaschen aufgefüllt wird.

### Bezugszeichenliste

- 1: erfindungsgemäße Abdeckung
- 2: Behälter
- 3: Flüssigkeit
- 4: Wandungen von 2
- 5: Hohl- bzw. Schwimmkörper
- 6: Gasraum
- 7: Hülle um 6
- 8: Oberwandung von 7
- 9: Unterwandung von 7
- 10: Seitenwandung von 7
- 11:
- 12: Gewicht
- 13:
- 14:
- 15:
- 16:
- 17:
- 18:
- 19:
- 20:
- 21:
- 22:
- 23:
- 24:
- 25:
- 26:
- 27:
- 28:
- 29: Beschwerungseinrichtung
- 30: Folienbahnen
- 31: freies Ende von 30
- 32: Gewicht
- 33: Kunststoffhohlkörper
- 34: Metallgewicht
- 35: durchgehender Folienstreifen
- 36: umlaufender Gewichtsring an 35
- 37: Entgasungslöcher
- 38:
- 39:
- 40:
- 41:
- 42:
- 43:
- 44:
- 101: Dach
- 102: Güllebehälter/Gasspeicher
- 103: Äußere elastische Folie
- 104: Gasableitung
- 105: Unterbau
- 106: Stütze
- 107: Seile/Sparren
- 108: Wetterschutzmembran
- 109: Innenmembran
- 110: Flüssigkeit
- 111: Gasraum
- 112: Membran
- 113: Holzring
- 114: Drucksensor
- 115: Gasableitung
- 116: Verdichter
- 117: Außenmembran
- 118: Wandungen des Behälters
- 119: Innenmembran
- 120: Rührer

## Patentansprüche

1. Abdeckung für nach oben offene Behälter, wie Güllebehälter oder Biogasspeicher, wobei die Abdeckung (1) sich flächig über einen abzudeckenden Behälterinhalt (3) erstrecken und auf diesem frei schwimmen kann, und die Abdeckung (1)ein aufblasbarer und luftmatratzenartig ausgebildeter Schwimmkörper (5) ist, der sich über die gesamte Fläche der Abdeckung erstreckt **dadurch gekennzeichnet, dass** der Schwimmkörper (5) durch das Aufblasen selbstständig entfaltbar ist und eine vorbestimmte Form und Eigensteifigkeit einnimmt, und der im nicht aufgeblasenen Zustand zusammmenfaltbar ist.

2. Abdeckung nach Anspruch 1, **dadurch gekennzeichnet, dass** im Bereich des Radiusmittelpunktes bzw. geometrischen Mittelpunktes eine nach außen weisende Öse vorhanden ist.

3. Abdeckung nach Anspruch 1 oder 2, **dadurch gekennzeichnet, dass** an dem Schwimmkörper (5) eine Pumpe angeschlossen ist.

4. Abdeckung nach einem der Ansprüche 1 bis 3, **dadurch gekennzeichnet, dass** außenseitig bzw. randseitig der Abdeckung (1) eine Beschwerungseinrichtung (29) vorhanden ist, welche die Abdeckung (1) auf dem Flüssigkeitsspiegel haltend ausgebildet ist.

5. Abdeckung nach einem der Ansprüche 1 bis 4, **dadurch gekennzeichnet, dass** die Abdeckung einen durchgehenden Folienstreifen (35) aufweist, der sich vom Umfang der Abdeckung nach unten erstreckt.

6. Abdeckung nach Anspruch 5, **dadurch gekennzeichnet, dass** am durchgehenden Folienstreifen (35) zumindest ein Entgasungsloch (37) vorgesehen ist, das vorzugsweise am unteren Randbereich des Folienstreifens (35) angeordnet ist.

7. Abdeckung nach Anspruch 5 oder 6, **dadurch gekennzeichnet, dass** der Folienstreifen als dicke Schwerfolie ausgebildet ist, wobei die Folie neben dem die Matrix bzw. tragende Struktur ausbildenden Kunststoff- bzw. Polymermaterial sogenannte Schwerzuschläge, insbesondere metallische Schwerzuschläge und/oder mineralische Schwerzuschläge, wie Eisenoxide und/oder Bariumsulfat oder ähnliche Schwerzuschläge enthält.

8. Abdeckung nach einem der Ansprüche 1 bis 7, **dadurch gekennzeichnet, dass** ein Überdruckspeicher vorhanden ist, der in der Lage ist, kurzfristig größere Luft- oder Gasmengen zum Aufblasen des Schwimmkörpers(5) zu liefern, um eine Notbetriebsfähigkeit der Abdeckung (1) bei einem Leck sicherzustellen.

9. Abdeckung nach einem der vorhergehenden Ansprüche, **dadurch gekennzeichnet, dass** zum Einstellen eines Spaltes zwischen dem Schwimmkörper (5), bzw. der Einrichtung (29) und der Behälterwandung (4) sowie zur Reibungsverminderung radial umfänglich an der Einrichtung (29) und/oder dem Schwimmkörper (5), Gleitschienen, die nach außen weisen, angeordnet sind.

10. Verfahren zum Abdecken eines Güllebehälters oder eines Biogasspeichers, wobei eine Abdeckung nach einem der Ansprüche 1 bis 9 verwendet wird, in dem
- die Abdeckung im zusammengefalteten bzw. zusammengelegten Zustand auf eine im Güllebehälter bzw. Biogasspeicher befindliche Flüssigkeit (3) aufgelegt wird,
- die Abdeckung aufblasbar ist und zum Abdecken der Flüssigkeit (3) entfaltet wird, und
- die Abdeckung sich durch Aufblasen des Schwimmkörpers (5) entfaltet bzw. ausrichtet und der Abdeckung (1) eine Eigensteifigkeit verleiht

## Claims

1. Cover for containers open at the top, such as liquid manure containers or biogas tanks, wherein the cover (1) extends flat over container contents (3) to be covered, and is able to float freely on top of them, and the cover (1) is an inflatable float (5) in the manner of an airbed extending over the whole area of the cover, **characterised in that** the float (5), because of the inflation, is able to unfold automatically and assume a predetermined form and inherent stiffness, and may be folded up in its non-inflated state.

2. Cover according to claim 1, **characterised in that** there is an outwards facing eyelet in the area of the radius centre point or geometrical centre point.

3. Cover according to claim 1 or 2, **characterised in that** a pump is connected to the float (5).

4. Cover according to any of claims 1 to 3, **characterised in that** the outside or edge of the cover (1) is provided with a weighting device (29) designed to hold the cover (1) on the liquid surface.

5. Cover according to any of claims 1 to 4, **characterised in that** the cover has a continuous sheet plastic strip (35) which extends downwards from the periphery of the cover.

6. Cover according to claim 5, **characterised in that** the continuous sheet plastic strip (35) is provided with at least one vent hole (37), located preferably in the lower edge section of the sheet plastic strip (35).

7. Cover according to claim 5 or 6, **characterised in that** the sheet plastic strip is in the form of a thick heavy plastic sheet, wherein the plastic sheet contains, in addition to the plastic or polymer material forming the matrix or supporting structure, heavy additives, in particular metallic, and/or mineral heavy additives, such as iron oxide and/or barium sulphate or similar heavy additives.

8. Cover according to any of claims 1 to 7, **characterised in that** there is a pressurised tank which is able to supply at short notice greater amounts of air or gas to inflate the float (5), in order to ensure that the cover (1) has an emergency operation capacity in the event of a leak.

9. Cover according to any of the preceding claims **characterised in that**, to create a gap between the float (5) and the device (29) on the one hand, and the container wall (4) on the other hand, and also to reduce friction, outwards facing slide rails are provided radiating from the periphery of the device (29) and/or the float (5).

10. Method of covering a liquid manure container or a biogas tank, wherein a cover according to any of claims 1 to 9 is used, in which:
- the cover is placed on a liquid (3) in the liquid manure container or biogas tank in the folded or collapsed state
- the cover may be inflated and unfolded to cover the liquid (3)
- the cover unfolds and aligns itself through inflation of the float (5), which gives the cover (1) an inherent stiffness.

## Revendications

1. Recouvrement pour des récipients ouverts vers le haut, comme des récipients à purin ou des accumulateurs de bio-gaz, dans lequel le recouvrement (1) s'étend en surface au-dessus du contenu d'un récipient (3) qu'il s'agit de recouvrir et peut flotter librement sur ce contenu, et le recouvrement (1) est un corps flottant gonflable (5) réalisé à la manière d'un matelas pneumatique, qui s'étend sur la totalité de la surface du recouvrement,
**caractérisé en ce que** le corps flottant (5) est automatiquement déployable par le gonflage et adopte une forme prédéterminée et une raideur propre prédéterminée, et **en ce qu'**il peut être replié sur lui-même dans l'état non gonflé.

2. Recouvrement selon la revendication 1, **caractérisé en ce qu'**il est prévu un oeillet tourné vers l'extérieur dans la zone du point au centre circulaire ou du point au centre géométrique.

3. Recouvrement selon la revendication 1 ou 2, **caractérisé en ce qu'**une pompe est raccordée au corps flottant (5).

4. Recouvrement selon l'une des revendications 1 à 3, **caractérisé en ce qu'**il est prévu un dispositif d'alourdissement (29) sur le côté extérieur ou sur le côté de la bordure du recouvrement (1), celui-ci étant réalisé de façon à maintenir le recouvrement (1) sur la surface du liquide.

5. Recouvrement selon l'une des revendications 1 à 4, **caractérisé en ce que** le recouvrement comprend une bande de feuille continue (35) qui s'étend vers le bas depuis la périphérie du recouvrement.

6. Recouvrement selon la revendication 5, **caractérisé en ce qu'**il est prévu au moins un trou de dégazage (37) dans la bande de feuille continue (35), ledit trou étant de préférence agencé au niveau de la zone de bordure inférieure de la bande de feuille (35).

7. Recouvrement selon la revendication 5 ou 6, **caractérisé en ce que** la bande de feuille est réalisée sous forme de feuille lourde épaisse, ladite feuille contenant, outre le matériau synthétique ou le matériau polymère qui forme la matrice, c'est-à-dire la structure portante, des additifs d'alourdissement, en particulier des additifs d'alourdissement métalliques et/ou des additifs d'alourdissements minéraux, comme de l'oxyde de fer et/ou du sulfate de baryum ou des additifs d'alourdissement similaires.

8. Recouvrement selon l'une des revendications 1 à 7, **caractérisé en ce qu'**il est prévu un accumulateur de surpression, qui est en mesure de fournir brièvement des grandes quantités d'air ou de gaz pour gonfler le corps flottant (5), afin de garantir une capacité fonctionnelle de secours du recouvrement (1) en cas de fuite.

9. Recouvrement selon l'une des revendications précédentes, **caractérisé en ce que**, pour établir une fente entre le corps flottant (5) ou le dispositif d'alourdissement (29) et la paroi (4) du récipient, ainsi que pour réduire les frictions, il est prévu des rails de coulissement tournés vers l'extérieur, agencés radialement à la périphérie sur le dispositif d'alourdissement (29) et/ou sur le corps flottant (5).

10. Procédé pour recouvrir un récipient à purin ou un récipient à bio-gaz, dans lequel on utilise un recouvrement selon l'une des revendications 1 à 9, dans lequel
- le recouvrement est posé, dans l'état replié ou rassemblé, sur un liquide (3) qui se trouve dans le récipient à purin ou le récipient à bio-gaz,
- le recouvrement est gonflable et est déployé pour recouvrir le liquide (3), et
- le recouvrement se déploie ou se redresse par gonflage du corps , flottant (5) et confère une propre rigidité au recouvrement (1).
